# EUROPEAN PATENT APPLICATION

(11) **EP 4 628 568 A1**
(43) Date of publication of application: **08.10.2025**
(21) Application number: 25159537.7
(22) Date of filing: 22.02.2025
(51) Int. Cl.: C11D 1/10, A61K 8/04, A61K 8/34, A61K 8/44, A61K 8/73, C11D 3/20, C11D 3/22, C11D 17/00

(54) **GEL CLEANING AGENT AND METHOD FOR PRODUCING THE SAME**

(30) Priority: 06.03.2024 JP 2024033879; 23.07.2024 JP 2024117406
(71) Applicant: Shiseido Honeycake Industries Co., Ltd., Ibaraki-shi, Osaka 567-0021 (JP)
(72) Inventor: NISHINA, Tetsuo, Osaka, 567-0021 (JP); HARADA, Koki, Osaka, 567-0021 (JP)
(74) Representative: Henkel & Partner mbB

(57) **Abstract**

Provided is a gel cleaning agent comprising (A) 0.8% by mass to 4.5% by mass of xyloglucan, (B) 7.5% by mass to 22.5% by mass of sodium lauroyl methylalanine, (C) 40% by mass to 50% by mass of polyhydric alcohol, and (D) 30% by mass to 35% by mass of water; wherein the polyhydric alcohol (C) comprises glycerin, and the amount of glycerin in the polyhydric alcohol is 80% by mass or more, and wherein the gel cleaning agent has a hardness at 25°C of 300 or more as measured by a rheometer.

## Description

### CROSS-REFERRENCE TO RELATED APPLICATIONS

The present application claims priority to Japanese Patent Application No. 2024-33879, filed March 6, 2024, and Japanese Patent Application No. 2024-117406, filed July 23, 2024, the entire contents of which are incorporated herein by reference.

### TECHNICAL FIELD

The present invention relates to a gel cleaning agent and a method for producing the gel cleaning agent.

### Description of the Related Art

### BACKGROUND ART

Conventionally, gel cleaning agents are known (e.g., Patent Literature 1 to Patent Literature 5). In all of these prior art documents, cleaning components are supported on gel using xyloglucan (hereinafter also referred to as "tamarind gum").

Further, Patent Literature 6 proposes a gel composition containing 2.0 parts by weight of tamarind gum, 30 parts by weight of glycerin, 25 parts by weight of soap base liquid, 2.0 parts by weight of decyl glucoside, 0.2 parts by weight of etidronic acid, 0.5 parts by weight of phenoxyethanol, 0.01 parts by weight of mannan, 10.0 parts by weight of ethanol, and 30.29 parts by weight of water, wherein a 1% by weight aqueous solution of the gel composition has a pH of less than 10.5.

Paragraph [0037] of Patent Literature 6 indicates that "examples of the soap base liquid include those prepared by mixing 10 parts by weight to 25 parts by weight of an aqueous solution (1) containing sodium lauroyl methylalanine (e.g., produced by Kawaken Fine Chemicals Co., Ltd., product name: Alanon ALE, concentration = 30% by weight) in an amount of 10% by weight of the total, 35 parts by weight to 50 parts by weight of an aqueous solution (2) containing a potash soap base (e.g., produced by Mighty K.K., concentration = 25% by weight) in an amount of 15% by weight of the total, 10 parts by weight to 30 parts by weight of coconut oil, 5 parts by weight to 15 parts by weight of potassium hydroxide (48% by weight aqueous solution), 0.01 parts by weight to 1 part by weight of methylparaben, and 5 parts by weight to 15 parts by weight of ethanol, stirring the resulting mixture while heating at 70°C to 90°C, and finally adding 0.01 parts by weight to 1 part by weight of etidronic acid to adjust the pH."

### Citation List

### Patent Literature

[Patent Literature 1] Japanese Patent Laid-Open No. 2019-116451
[Patent Literature 2] Japanese Patent Laid-Open No. 2020-100588
[Patent Literature 3] Japanese Patent Laid-Open No. 2020-189956
[Patent Literature 4] Japanese Patent Laid-Open No. 2021-102600
[Patent Literature 5] Japanese Patent Laid-Open No. 2018-062525
[Patent Literature 6] Japanese Patent Laid-Open No. 2016-216664

### SUMMARY OF THE INVENTION

### TECHNICAL PROBLEM

All of the gel cleaning agents described in Patent Literature 1 to Patent Literature 5 contain xyloglucan (tamarind gum), which was difficult to dissolve uniformly using conventional formulations and production methods, resulting in reduction in syneresis properties and foaming properties, as well as tearing or blistering during use in some cases.

In Patent Literature 6 mentioned above, a soap base liquid containing sodium lauroyl methylalanine is used. The soap base liquid is described such that "in the aqueous solution containing sodium lauroyl methylalanine (e.g., produced by Kawaken Fine Chemicals Co., Ltd., product name: Alanon ALE, concentration = 30% by weight) in an amount of 10% by weight of the total, 10% by weight of sodium lauroyl methylalanine (produced by Kawaken Fine Chemicals Co., Ltd., product name: Alanon ALE, concentration = 30% by weight) is incorporated" (see paragraph [0037] of Patent Literature 6). Since this sodium lauroyl methylalanine aqueous solution contains 30% by weight of sodium lauroyl methylalanine, the content of sodium lauroyl methylalanine in the sodium lauroyl methylalanine aqueous solution is calculated to be 0.3 parts by weight to 0.75 parts by weight (0.46% by weight to 0.55% by weight). In all of Examples 1 to 9 of Patent Literature 6, 25 parts by weight of soap base liquid containing sodium lauroyl methylalanine is incorporated; thus, the content of sodium lauroyl methylalanine in the gel compositions of Examples 1 to 9 of Patent Literature 6 is 0.12% by weight to 0.14% by weight, which is a very small amount compared to the content of sodium lauroyl methylalanine in the present invention, i.e., 7.5% by mass to 22.5% by mass. It is impossible to provide a gel cleaning agent having excellent appearance (transparency), hardness, and foaming properties.

### SOLUTION TO PROBLEM

The present invention was made in view of the above circumstances, and an object thereof is to provide a gel cleaning agent having excellent transparency, hardness, syneresis properties, and usability, as well as a method for producing the gel cleaning agent having excellent production suitability.

The gel cleaning agent of the present invention as a means for solving the problems contains (A) 0.8% by mass to 4.5% by mass of xyloglucan, (B) 7.5% by mass to 22.5% by mass of sodium lauroyl methylalanine, (C) 40% by mass to 50% by mass of polyhydric alcohol, and (D) 30% by mass to 35% by mass of water; wherein the polyhydric alcohol (C) contains glycerin, and the amount of glycerin in the polyhydric alcohol is 80% by mass or more, and wherein the gel cleaning agent has a hardness at 25°C of 300 or more as measured by a rheometer.

Further, the gel cleaning agent preferably contains (A) 1% by mass to 3% by mass of xyloglucan, (B) 10% by mass to 15% by mass of sodium lauroyl methylalanine, and (C) 42% by mass to 47% by mass of polyhydric alcohol.

Further, a 1% by mass aqueous solution of the gel cleaning agent preferably has a pH of 7.5 or less.

Further, in the gel cleaning agent, the polyhydric alcohol (C) preferably further contains propanediol.

Further, in the gel cleaning agent, the content of propanediol is preferably 6% by mass to 9% by mass.

Further, the gel cleaning agent is preferably transparent.

The method for producing the gel cleaning agent of the present invention is a method for producing the gel cleaning agent of the present invention, the method including a cooling solidification step of cooling and solidifying a composition in which (A') 0.5% by mass to 3% by mass of xyloglucan, (B') 5% by mass to 15% by mass of sodium lauroyl methylalanine, (C') 20% by mass to 40% by mass of polyhydric alcohol, and (D') 40% by mass to 60% by mass of water are dissolved, and an aging step of aging a gel composition after cooling and solidification in a constant temperature and humidity chamber for one week or more to remove a solvent by evaporation from the gel composition; wherein the polyhydric alcohol (C') contains glycerin, and the amount of glycerin in the polyhydric alcohol is 80% by mass or more.

Further, in the method for producing the gel cleaning agent, the polyhydric alcohol (C') preferably further contains propanediol.

Further, in the method for producing the gel cleaning agent, the content of propanediol is preferably 5% by mass to 10% by mass.

### ADVANTAGEOUS EFFECTS OF THE INVENTION

The present invention can solve the problems in the past and can achieve the object, and its object is to provide a gel cleaning agent having excellent transparency, hardness, syneresis properties, and usability, as well as a method for producing the gel cleaning agent with excellent production suitability.

### DETAILED DESCRIPTION OF THE INVENTION

### (Gel cleaning agent)

The gel cleaning agent of the present invention contains (A) xyloglucan, (B) sodium lauroyl methylalanine, (C) polyhydric alcohol, and (D) water, and further contains other components, as necessary.

### <(A) Xyloglucan (tamarind gum)>

Xyloglucan is the main component of seeds of *Tamarindus indica,* which is a leguminous plant mainly found in tropical regions.

Xyloglucan is a polysaccharide with a molecular weight of hundreds of thousands having a structure represented by the following formula (1) in which xylose as a side chain is linked via an **α**-1,6 bond to a part of the main chain consisting of **β**-1,4-glucan, and galactose is linked to a part of xylose. 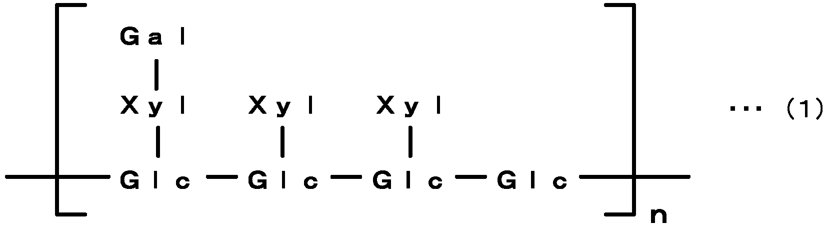 In the formula (1), Glc represents glucose, Xyl represents xylose, Gal represents galactose, and n represents the number of bonds.

The content of xyloglucan is 0.8% by mass to 4.5% by mass, preferably 1% by mass to 4% by mass, and more preferably 1% by mass to 3% by mass, based on the total amount of the gel cleaning agent. If the content of xyloglucan is less than 0.8% by mass, the gel cleaning agent softens, making it difficult to maintain its shape. On the other hand, if the content of xyloglucan exceeds 4.5% by mass, the composition has an increased viscosity and may not be uniformly dissolved.

Usable examples of xyloglucan include commercially available xyloglucan (product name "Glyloid 6C," manufactured by Dainippon Pharmaceutical Co., Ltd.). When a commercial product is incorporated into the gel cleaning agent of the present invention, it is preferable to use one that is as purified as possible. The molecular weight of xyloglucan used is not particularly limited.

The gel cleaning agent of the present invention can be incorporated with xyloglucan produced by a known method, for example, the method listed in the product catalog of "Glyloid 6C" mentioned above. Specifically, xyloglucan can be produced by removing foreign matter from tamarind seeds, dissolving the seeds in water, removing impurities, and making them transparent, followed by drying and pulverization.

### <(B) Sodium lauroyl methylalanine>

Sodium lauroyl methylalanine can impart excellent appearance (transparency), hardness, and foaming properties to the gel cleaning agent.

Sodium lauroyl methylalanine is a sodium salt of a condensate of lauric acid and N-methyl-**β**-alanine represented by the following chemical formula:

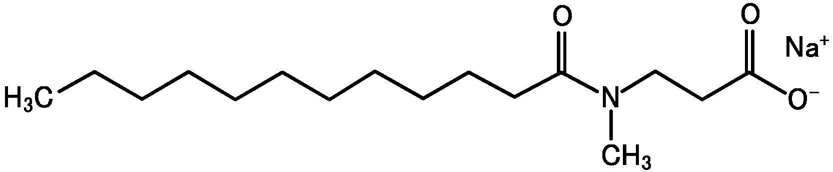

The content of sodium lauroyl methylalanine is 7.5% by mass to 22.5% by mass, preferably 10% by mass to 20% by mass, and more preferably 10% by mass to 15% by mass, based on the total amount of the gel cleaning agent. If the content of sodium lauroyl methylalanine is less than 7.5% by mass, cleaning power and foaming may be insufficient. On the other hand, if the content of sodium lauroyl methylalanine exceeds 22.5% by mass, the gel structure of the gel cleaning agent collapses, and the gel state may not be maintained.

### <(C) Polyhydric alcohol>

The polyhydric alcohol contains glycerin, and the amount of glycerin in the polyhydric alcohol is 80% by mass or more.

The polyhydric alcohol preferably further contains propanediol. The content of propanediol is preferably 5% by mass to 10% by mass, more preferably 6% by mass to 9% by mass, and even more preferably 7% by mass to 8% by mass.

As the polyhydric alcohol, for example, diglycerin, xylitol, trehalose, and the like can be used, in addition to glycerin and propanediol.

The content of the polyhydric alcohol is 40% by mass to 50% by mass, and preferably 42% by mass to 47% by mass, based on the total amount of the gel cleaning agent. If the content of the polyhydric alcohol is less than 40% by mass, the hardness of the gel cleaning agent may be reduced. On the other hand, if the content of the polyhydric alcohol exceeds 50% by mass, the gel composition is thickened, and it may be difficult to uniformly dissolve the gel composition.

### <(D) Water>

Usable water is water for use in cosmetics, quasi-drugs, or the like, and examples thereof include ion exchange water, ultrafiltered water, reverse osmosis water, distilled water, purified water, pure water, ultrapure water, tap water, and the like.

The content of water is 30% by mass to 35% by mass based on the total amount of the gel cleaning agent. If the content of water is less than 30% by mass, there is less dissolving loss, and stickiness tends to occur. If the content of water exceeds 35% by mass, significant syneresis occurs, and tearing or blistering may occur during use.

### <Other components>

The gel cleaning agent of the present invention may contain other components within the range in which the object and effects of the present invention are not impaired.

Examples of other components include anionic surfactants, cationic surfactants, amphoteric surfactants, nonionic surfactants, moisturizers, water-soluble polymers, thickeners, coating agents, UV absorbers, sequestering agents, lower alcohols, oil components, sugars, amino acids, organic amines, polymer emulsions, pH adjusters, skin nutrients, vitamins, antioxidants, antioxidant aids, fragrances, colorants, preservatives, disinfectants, and the like, which are appropriately incorporated as necessary, thereby producing a gel cleaning agent according to the desired dosage form.

Examples of anionic surfactants include higher alkyl sulfate ester salts (e.g., sodium lauryl sulfate and potassium lauryl sulfate); alkyl ether sulfate ester salts (e.g., POE-lauryl sulfate triethanolamine and POE-sodium lauryl sulfate); N-acyl sarcosinates (e.g., sodium lauroyl sarcosine); higher fatty acid amide sulfonates (e.g., N-myristoyl-N-methyl taurine sodium, coconut oil fatty acid methyl taurine sodium, and lauroyl methyl taurine sodium); phosphate ester salts (sodium POE-oleyl ether phosphate, POE-stearyl ether phosphate, etc.); sulfosuccinates (e.g., sodium di-2-ethylhexyl sulfosuccinate, sodium monolauroyl monoethanolamide polyoxyethylene sulfosuccinate, and sodium lauryl polypropylene glycol sulfosuccinate); alkylbenzene sulfonates (e.g., sodium linear dodecylbenzenesulfonate, and linear dodecylbenzenesulfonate triethanolamine); higher fatty acid ester sulfate ester salts (e.g., hydrogenated coconut oil fatty acid sodium glycerin sulfate); N-acyl glutamates (e.g., monosodium N-lauroylglutamate, disodium N-stearoylglutamate, and monosodium N-myristoyl-L-glutamate); acylglycine salts, such as cocoyl glycine salt and lauroyl glycine salt; sulfated oils (e.g., sulfonated castor oil); acylalanine salts, such as lauroyl methylalanine salt; POE-alkyl ether carboxylic acids; POE-alkyl allyl ether carboxylates; α-olefin sulfonates; higher fatty acid ester sulfonates; secondary alcohol sulfate ester salts; higher fatty acid alkylolamide sulfate ester salts; sodium lauroyl monoethanolamide succinate; ditriethanolamine N-palmitoylaspartate; sodium caseinate, and the like.

Examples of cationic surfactants include alkyltrimethylammonium salts (e.g., stearyltrimethylammonium chloride and behenyltrimethylammonium chloride); alkylpyridinium salts (e.g., cetylpyridinium chloride); distearyldimethylammonium chloride and dialkyldimethylammonium salts; poly(N,N'-dimethyl-3,5-methylene piperidinium) chloride; alkyl quaternary ammonium salts; alkyldimethylbenzylammonium salts; alkylisoquinolinium salts; dialkylmorphonium salts; POE-alkyl amines; alkyl amine salts; polyamine fatty acid derivatives; amyl alcohol fatty acid derivatives; benzalkonium chloride; benzethonium chloride; and the like.

Examples of amphoteric surfactants include imidazoline-based amphoteric surfactants (e.g., 2-undecyl-N,N,N-(hydroxyethylcarboxymethyl)-2-imidazoline sodium, 2-cocoyl-2-imidazolinium hydroxide-1-carboxyethyloxy disodium salt, and 2-heptadecyl-N-carboxymethyl-N-hydroxyethylimidazolinium betaine); betaine-based surfactants (e.g., lauryldimethylaminoacetic acid betaine, alkyl betaine, alkyl amide betaine, and alkyl sulfobetaine); and the like.

Examples of lipophilic nonionic surfactants include sorbitan fatty acid esters (e.g., sorbitan monooleate, sorbitan monoisostearate, sorbitan monolaurate, sorbitan monopalmitate, sorbitan monostearate, sorbitan sesquioleate, sorbitan trioleate, diglycerol sorbitan penta-2-ethylhexylate, and diglycerol sorbitan tetra-2-ethylhexylate); glycerin polyglycerin fatty acids (e.g., mono cottonseed oil fatty acid glycerin, glycerin monoerucate, glycerin sesquioleate, glycerin monostearate, glycerin **α,α'**-oleate pyroglutamate, and monostearate glycerin malate); propylene glycol fatty acid esters (e.g., propylene glycol monostearate); hydrogenated castor oil derivatives; glycerin alkyl ethers, and the like.

Examples of hydrophilic nonionic surfactants include POE-sorbitan fatty acid esters (e.g., POE-sorbitan monooleate, POE-sorbitan monostearate, and POE-sorbitan tetraoleate); POE-sorbitol fatty acid esters (e.g., POE-sorbitol monolaurate, POE-sorbitol monooleate, POE-sorbitol pentaoleate, and POE-sorbitol monostearate); POE-glycerin fatty acid esters (e.g., POE-monooleates, such as POE-glycerin monostearate, POE-glycerin monoisostearate, and POE-glycerin triisostearate); POE-fatty acid esters (e.g., POE-distearate, POE-monodioleate, and ethylene glycol distearate); POE-alkyl ethers (e.g., POE-lauryl ether, POE-oleyl ether, POE-stearyl ether, POE-behenyl ether, POE-2-octyldodecyl ether, and POE-cholestanol ether); Pluronic (registered trademark) series; POE·POP-alkyl ethers (e.g., POE·POP-cetyl ether, POE·POP-2-decyltetradecyl ether, POE·POP-monobutyl ether, POE·POP-hydrogenated lanolin, and POE·POP-glycerin ether); tetra-POE/tetra-POP-ethylenediamine condensates (e.g., Tetronic); POE-castor oil hydrogenated castor oil derivatives (e.g., POE-castor oil, POE-hydrogenated castor oil, POE-hydrogenated castor oil monoisostearate, POE-hydrogenated castor oil triisostearate, POE-hydrogenated castor oil monopyroglutamate monoisostearate diester, and POE-hydrogenated castor oil maleate); POE-beeswax lanolin derivatives (e.g., POE-sorbitol beeswax); alkanolamides (e.g., coconut oil fatty acid diethanolamide, lauric acid monoethanolamide, fatty acid isopropanolamide, and cocamide methyl monoethanolamide); POE-propylene glycol fatty acid esters; POE-alkylamines; POE-fatty acid amides; diethylene glycol laurate; sucrose fatty acid esters; alkylethoxydimethylamine oxides; trioleyl phosphate; and the like.

Examples of moisturizers include polyethylene glycol, propylene glycol, dipropylene glycol, glycerin, 1,3-butylene glycol, xylitol, sorbitol, maltitol, chondroitin sulfate, hyaluronic acid, mucoitin sulfate, caronic acid, atelocollagen, cholesteryl-12-hydroxystearate, sodium lactate, bile salts, dl-pyrrolidone carboxylate, alkylene oxide derivatives, short-chain soluble collagen, diglycerin (EO) PO adducts, *Rosa roxburghii* extract, yarrow extract, melilot extract, and the like.

Examples of natural water-soluble polymers include plant-based polymers (e.g., gum arabic, tragacanth gum, galactan, guar gum, carob gum, karaya gum, carrageenan, pectin, agar, quince seed (marmelo), algae colloid (brown alga extract), starches (rice, corn, potato, and wheat), and glycyrrhizic acid); microorganism-based polymers (e.g., xanthan gum, dextran, succinoglycan, and pullulan); animal-based polymers (e.g., collagen, casein, albumin, and gelatin); and the like.

Examples of semi-synthetic water-soluble polymers include starch-based polymers (e.g., carboxymethyl starch and methylhydroxypropyl starch); cellulose-based polymers (methylcellulose, ethylcellulose, methylhydroxypropylcellulose, hydroxyethylcellulose, sodium cellulose sulfate, hydroxypropylcellulose, carboxymethylcellulose, carboxymethylcellulose sodium, crystalline cellulose, and cellulose powder); alginic acid-based polymers (e.g., sodium alginate and alginate propylene glycol ester); and the like.

Examples of synthetic water-soluble polymers include vinyl-based polymers (e.g., polyvinyl alcohol, polyvinyl methyl ether, polyvinylpyrrolidone, and carboxyvinyl polymer); polyoxyethylene-based polymers (e.g., polyoxyethylene polyoxypropylene copolymers of polyethylene glycol 20,000, 40,000, or 60,0000, and high polymer polyethylene glycol); acrylic polymers (e.g., sodium polyacrylate, polyethyl acrylate, and polyacrylamide); polyethyleneimine; cationic polymers, and the like.

Examples of thickeners include gum arabic, carrageenan, karaya gum, tragacanth gum, carob gum, quince seed (marmelo), casein, dextrin, gelatin, sodium pectate, sodium alginate, methylcellulose, ethylcellulose, CMC, hydroxyethylcellulose, hydroxypropylcellulose, PVA, PVM, PVP, sodium polyacrylate, carboxyvinyl polymer, locust bean gum, guar gum, tamarind gum, dialkyldimethyl ammonium sulfate cellulose, xanthan gum, magnesium aluminum silicate, bentonite, hectorite, Al-Mg silicate (Veegum), laponite, silicic anhydride, and the like.

Examples of UV absorbers include benzoic acid-based UV absorbers (e.g., para-aminobenzoic acid (hereinafter abbreviated as PABA), PABA monoglycerin ester, N,N-dipropoxy PABA ethyl ester, N,N-diethoxy PABA ethyl ester, N,N-dimethyl PABA ethyl ester, N,N-dimethyl PABA butyl ester, and N,N-dimethyl PABA ethyl ester); anthranilic acid-based UV absorbers (e.g., homomenthyl-N-acetylanthranilate); salicylic acid-based UV absorbers (e.g., amyl salicylate, menthyl salicylate, homomenthyl salicylate, octyl salicylate, phenyl salicylate, benzyl salicylate, and p-isopropanol phenylsalicylate); cinnamic acid-based UV absorbers (e.g., octylmethoxycinnamate, ethyl-4-isopropylcinnamate, methyl-2,5-diisopropylcinnamate, ethyl-2,4-diisopropylcinnamate, methyl-2,4-diisopropylcinnamate, propyl-p-methoxycinnamate, isopropyl-p-methoxycinnamate, isoamyl-p-methoxycinnamate, octyl-p-methoxycinnamate(2-ethylhexyl-p-methoxycinnamate), 2-ethoxyethyl-p-methoxycinnamate, cyclohexyl-p-methoxycinnamate, ethyl-**α-**cyano-**β**-phenylcinnamate, 2-ethylhexyl-**α**-cyano-**β-**phenylcinnamate, and glyceryl mono-2-ethylhexanoyl-diparamethoxycinnamate); benzophenone-based UV absorbers (e.g., 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone, 2-hydroxy-4-methoxy-4'-methylbenzophenone, 2-hydroxy-4-methoxybenzophenone-5-sulfonate, 4-phenylbenzophenone, 2-ethylhexyl-4'-phenylbenzophenone-2-carboxylate, 2-hydroxy-4-n-octoxybenzophenone, and 4-hydroxy-3-carboxybenzophenone); 3-(4'-methylbenzylidene)-d,l-camphor, 3-benzylidene-d,l-camphor; 2-phenyl-5-methylbenzoxazole; 2,2'-hydroxy-5-methylphenylbenzotriazole; 2-(2'-hydroxy-5'-t-octylphenyl)benzotriazole; 2-(2'-hydroxy-5'-methylphenylbenzotriazole; dibenzalazine; dianisoylmethane; 4-methoxy-4'-t-butyldibenzoylmethane; 5-(3,3-dimethyl-2-norbornylidene)-3-pentan-2-one, dimorpholinopyridazinone; 2-ethylhexyl-2-cyano-3,3-diphenylacrylate; 2,4-bis-{[4-(2-ethylhexyloxy)-2-hydroxy]-phenyl}-6-(4-methoxyphenyl)-(1,3,5)-triazine; and the like.

Examples of sequestering agents include 1-hydroxyethane-1,1-diphosphonic acid, 1-hydroxyethane-1,1-diphosphonic acid tetrasodium salt, disodium edetate, trisodium edetate, tetrasodium edetate, sodium citrate, sodium polyphosphate, sodium metaphosphate, gluconic acid, phosphoric acid, citric acid, ascorbic acid, succinic acid, edetic acid, trisodium ethylenediaminehydroxyethyl triacetate, and the like.

Examples of lower alcohols include ethanol, propanol, isopropanol, isobutyl alcohol, t-butyl alcohol, and the like.

Examples of monosaccharides include trioses (e.g., D-glyceryl aldehyde and dihydroxyacetone); tetroses (e.g., D-erythrose, D-erythrulose, D-threose, and erythritol); pentoses (e.g., L-arabinose, D-xylose, L-lyxose, D-arabinose, D-ribose, D-ribulose, D-xylulose, and L-xylulose); hexoses (e.g., D-glucose, D-talose, D-psicose, D-galactose, D-fructose, L-galactose, L-mannose, and D-tagatose); heptoses (e.g., aldoheptose and heptose); octoses (e.g., octulose); deoxy sugars (e.g., 2-deoxy-D-ribose, 6-deoxy-L-galactose, and 6-deoxy-L-mannose); amino sugars (e.g., D-glucosamine, D-galactosamine, sialic acid, aminouronic acid, and muramic acid); uronic acids (e.g., D-glucuronic acid, D-mannuronic acid, L-guluronic acid, D-galacturonic acid, and L-iduronic acid); and the like.

Examples of oligosaccharides include sucrose, gentianose, umbelliferose, lactose, planteose, isolychnose, **α**,**α**-trehalose, raffinose, lychnose, umbilicin, stachyose verbascose, and the like.

Examples of polysaccharides include cellulose, quince seed, chondroitin sulfate, starch, galactan, dermatan sulfate, glycogen, gum arabic, heparan sulfate, hyaluronic acid, tragacanth gum, keratan sulfate, chondroitin, mucoitin sulfate, guar gum, dextran, keratosulfate, locust bean gum, succinoglycan, caronic acid, and the like.

Examples of amino acids include neutral amino acids (e.g., threonine and cysteine); basic amino acids (e.g., hydroxylysine); and the like. Examples of amino acid derivatives include acyl sarcosine sodium (sodium lauroyl sarcosine), acyl glutamate, sodium acyl **β**-alanine, glutathione, pyrrolidonecarboxylic acid, and the like.

Examples of organic amines include monoethanolamine, diethanolamine, triethanolamine, morpholine, triisopropanolamine, 2-amino-2-methyl-1,3-propanediol, 2-amino-2-methyl-1-propanol, and the like.

Examples of polymer emulsions include acrylic resin emulsion, polyacrylic acid ethyl emulsion, acrylic resin liquid, polyacrylic alkyl ester emulsion, polyvinyl acetate resin emulsion, natural rubber latex, and the like.

Examples of pH adjusters include buffers, such as citric acid, lactic acid-sodium lactate, citric acid-sodium citrate, and succinic acid-sodium succinate.

Examples of vitamins include vitamins A, B₁, B₂, B₆, C, and E, or derivatives thereof, pantothenic acid or derivatives thereof, biotin, and the like.

Examples of antioxidants include tocopherols, dibutylhydroxytoluene, butylhydroxyanisole, gallic acid esters, and the like.

Examples of antioxidant aids include phosphoric acid, citric acid, ascorbic acid, maleic acid, malonic acid, succinic acid, fumaric acid, cephalin, hexametaphosphate, phytic acid, ethylenediaminetetraacetic acid, and the like.

Examples of other components that can be incorporated include preservatives (ethylparaben, butylparaben, chlorphenesin, phenoxyethanol, etc.); disinfectants (e.g., isopropyl methylphenol, benzalkonium chloride, benzethonium chloride, and chlorhexidine gluconate); antiphlogistics (e.g., glycyrrhizic acid derivatives, glycyrrhetinic acid derivatives, salicylic acid derivatives, hinokitiol, zinc oxide, and allantoin); whitening agents (e.g., placenta extract, saxifrage extract, and arbutin); various extracts (e.g., phellodendron bark, *Coptis japonica*, lithospermum root, peony root, *Swertia japonica*, birch, sage, loquat, carrot, aloe, mallow, iris, grape, coix seed, loofah, lily, saffron, cnidium rhizome, ginger, hypericum, ononis, garlic, chili pepper, chenpi, Japanese angelica root, and seaweed), activators (e.g., royal jelly, photosensitizers, and cholesterol derivatives); blood circulation enhancers (e.g., vanillylamide nonylate, nicotinic acid benzyl ester, nicotinic acid **β**-butoxyethyl ester, capsaicin, zingerone, cantharis tincture, ichthammol, tannic acid, **α**-borneol, tocopherol nicotinate, inositol hexanicotinate, cyclandelate, cinnarizine, tolazoline, acetylcholine, verapamil, cepharanthine, and **γ**-oryzanol); antiinflammatory agents (e.g., tranexamic acid, thiotaurine, and hypotaurine); sequestering agents, such as disodium edetate, trisodium edetate, sodium citrate, sodium polyphosphate, sodium metaphosphate, gluconic acid, and malic acid; various crude drug extracts, such as caffeine, tannin, verapamil, tranexamic acid or derivatives thereof, licorice, quince, and shinleaf; drugs, such as tocopherol acetate; whitening agents, such as vitamin C, magnesium ascorbate phosphate, glucoside ascorbate, arbutin, and kojic acid; amino acids or derivatives thereof, such as arginine and lysine; sugars, such as fructose, mannose, erythritol, trehalose, and xylitol; and the like.

The hardness at 25°C of the gel cleaning agent of the present invention as measured by a rheometer is 300 or more, preferably 300 or more and 600 or less, and more preferably 350 or more and 500 or less. When the hardness at 25°C as measured by a rheometer is 300 or more, the gel cleaning agent has sufficient hardness.

The hardness at 25°C as measured by a rheometer can be measured, for example, using a rheometer to which a needle with a diameter of 2 mm is attached under the conditions of 25°C, a penetration speed of 2 cm/min, and a penetration distance of 10 mm.

The pH of a 1% by mass aqueous solution of the gel cleaning agent of the present invention is preferably 7.5 or less, and more preferably 6.0 to 7.5, in terms of irritation to the skin and foaming properties.

The pH of the 1% by mass aqueous solution is the pH value of a 1% by mass aqueous solution prepared by dissolving the gel cleaning agent and water at a ratio of 1:99, as measured by a pH meter.

The gel cleaning agent of the present invention is preferably transparent. The appearance (transparency) of the gel cleaning agent can be evaluated by visual observation.

### (Method for producing gel cleaning agent)

The method for producing the gel cleaning agent of the present invention includes a cooling solidification step of cooling and solidifying a composition in which (A') 0.5% by mass to 3% by mass of xyloglucan, (B') 5% by mass to 15% by mass of sodium lauroyl methylalanine, (C') 20% by mass to 40% by mass of polyhydric alcohol, and (D') 40% by mass to 60% by mass of water are dissolved, and an aging step of aging a gel composition after cooling and solidification in a constant temperature and humidity chamber for one week or more to remove a solvent by evaporation from the gel composition, and further includes other steps, as necessary.

The method for producing the gel cleaning agent of the present invention has the advantage that since a large amount of solvent, such as water or polyhydric alcohol, is contained during production, components that have been difficult to incorporate in conventional production methods can be dissolved uniformly, performing the aging step allows the moderate evaporation of the solvent, such as water, and a gel cleaning agent that has high hardness, is less likely to undergo syneresis, and has excellent transparency and usability can be obtained.

The amount of xyloglucan incorporated during production is preferably 0.5% by mass to 3% by mass because a gel-like state can be obtained, and the amount of xyloglucan is more preferably 1% by mass to 2% by mass. A xyloglucan content of less than 0.5% by mass may be undesirable because the cleaning agent does not gel. On the other hand, a xyloglucan content of more than 3% by mass may be undesirable because the solubility of the gel cleaning agent is reduced.

The amount of sodium lauroyl methylalanine incorporated during production is preferably 5% by mass to 15% by mass in the composition. A sodium lauroyl methylalanine content of less than 5% by mass may be undesirable because of poor cleaning properties. On the other hand, a sodium lauroyl methylalanine content of more than 15% by mass may be undesirable in terms of compatibility with other components and production suitability.

The polyhydric alcohol (C') contains glycerin, and the amount of glycerin in the polyhydric alcohol is 80% by mass or more.

The polyhydric alcohol preferably further contains propanediol. The content of propanediol is preferably 5% by mass to 10% by mass.

Since 40% by mass or more of solvent (water) is required during production, the amount of the polyhydric alcohol incorporated is 20% by mass to 40% by mass in the gel composition, in terms of production suitability.

The amount of water incorporated during production is 40% by mass to 60% by mass in the gel composition.

Water evaporates through the aging step, and the content of water after aging is 30% by mass to 35% by mass in the composition.

The method for producing the gel cleaning agent of the present invention utilizes a frame kneading production method in which a solvent, such as water or alcohol, is contained during production, various components are uniformly dissolved, and after cooling and solidification, the solvent, such as water, is evaporated by aging. Specifically, water, polyhydric alcohol, and the like are placed in a production pot, and dissolved by heating, after which xyloglucan is added and dissolved by stirring. Next, a surfactant etc. are added, and the mixture is uniformly mixed and cooled.

After that, since a relatively large amount of solvent (water) is used as a preparation component, after cooling, the solvent is removed by evaporation in a constant temperature and humidity chamber for one week or more, preferably one week to three months, and more preferably one month to three months, which is the so-called aging step.

The method for producing the gel cleaning agent of the present invention has the advantage that through a step of aging an unaged gel composition, a gel cleaning agent that has increased hardness and is less likely to undergo syneresis can be obtained.

### Examples

Examples of the present invention will be described below; however, the present invention is not limited to these Examples.

In the following Examples, as a method for calculating the mass of each component after aging, first, the total weight of the composition after aging was weighed. Since the components other than water, which is a volatile solvent, were not volatile components, or their volatility was extremely low, calculation was made on the assumption that the weight loss due to aging was regarded as the content of water evaporated. In the following Examples, evaluation was performed using the following methods.

### (1) Appearance (transparency)

The appearance (transparency) at room temperature (25°C) of gel compositions before the aging step and passed 24 hours after preparation, and gel cleaning agents after the aging step was visually observed and evaluated based on the following evaluation criteria.

### [Evaluation criteria]

A: Transparent
B: Slightly cloudy
C: Translucent
D: Opaque
-: Liquefied

### (2) Hardness

The hardness of gel compositions before the aging step and passed 24 hours after preparation, and gel cleaning agents after the aging step was measured using a rheometer (CR-100, manufactured by Sun Scientific Co., Ltd.) to which a needle with a diameter of 2 mm was attached under the conditions of 25°C, a penetration speed of 2 cm/min, and a penetration distance of 10 mm.

### [Evaluation criteria of hardness of gel compositions before aging step]

A: 50 or more and 150 or less
B: 10 or more and less than 50
C: 1 or more and less than 10
D: 0

### [Evaluation criteria of hardness of gel cleaning agents after aging step]

A: 300 or more
B: 100 or more and less than 300
C: 1 or more and less than 100
D: 0

### (3) Foaming properties

The liquid amount (ml) was determined after 400 ml of a 2% by mass aqueous solution of the gel cleaning agent was stirred at 25°C at a maximum stirring number of 4,300 rpm for 1 minute, and foaming properties were evaluated based on the following evaluation criteria.

### [Evaluation criteria]

A: More than 2,000 ml
B: 1,500 ml or more and 2,000 ml or less
C: 1,000 ml or more and less than 1,500 ml
D: Less than 1,000 ml

### (4) Syneresis properties

The gel cleaning agent was cut into cubic specimens of about 2 cm on each side, and the specimens were sealed in screw tubes and allowed to stand in an incubator at 25°C for one week. The specimens were then taken out, the syneresis rate was calculated from the change in weight before and after standing for one week, and syneresis properties were evaluated based on the following evaluation criteria.

### [Evaluation criteria]

A: 0.1% or more
B: More than 0.1% and 1% or less
C: More than 1% and 5% or less
D: More than 5% and 10% or less
E: More than 10%

### (5) Production suitability (viscosity)

Production suitability (viscosity) during the production of the gel cleaning agents was evaluated based on the following evaluation criteria

### [Evaluation criteria]

A: No problem
B: Slightly thickened
C: Thickened
D: Unable to be produced

### (6) Usability

Both hands were wetted with warm water at 40°C, and the gel cleaning agent was rolled 10 times between the palms of both hands, then foamed, and used. Tearing, blistering, dissolving loss, stickiness, etc. were checked during use, and usability was evaluated based on the following evaluation criteria.

### [Evaluation criteria]

A: No problem
B: Somewhat problematic (slightly tearing, slightly blistering, little dissolving loss, slightly sticky)
C: Problematic (tearing, blistering, less melting, sticky)

### (Test Examples 1-1 to 1-10)

Using xyloglucan (tamarind gum), gel compositions before the aging step and gel cleaning agents were produced, and suitable anionic surfactants were examined for transparency, foaming properties, and hardness.

According to the formulations shown in Table 1-1 to Table 1-4, water, polyhydric alcohol, etc. were placed in a production pot and dissolved by heating, after which xyloglucan was added and dissolved by stirring. Thereafter, a surfactant etc. were added, and the mixture was uniformly mixed and cooled (cooling solidification step).

Because a relatively large amount of water was used as a solvent as a preparation component, after cooling, the solvent was removed by evaporation in a constant temperature and humidity chamber for 1 month to 2 months of aging period (aging step), thereby obtaining gel cleaning agents.

Next, the appearance (transparency), hardness, and foaming properties were evaluated before and after the aging step. The results are shown in Tables 1-1 to 1-4.

**[Table 1-1]**

| Classificati on | Component name | Test Example | | | | | |
|---|---|---|---|---|---|---|---|
| | | 1-1 | 1-1 (aging) | 1-2 | 1-2 (aging) | 1-3 | 1-3 (aging) |
| Anionic SAA | Lauryl glycol carboxylate Na | 7.5 | 11.2 | - | - | - | - |
| | Cocoyl methyl taurine Na | - | - | 7.5 | 11.2 | - | - |
| | Cocoyl methyl taurine taurine Na | - | - | - | - | 7.5 | 11.2 |
| | Lauroyl methylalanine Na | - | - | - | - | - | - |
| | Lauroyl-β-alanine Na | - | - | - | - | - | - |
| Amphoteric SAA | Cocamidopropyl betaine | 4.0 | 6.0 | 4.0 | 6.0 | 4.0 | 6.0 |
| Nonionic SAA | Cocamide methyl MEA | 2.0 | 3.0 | 2.0 | 3.0 | 2.0 | 3.0 |
| Gelling agent | Tamarind gum | 1.5 | 2.2 | 1.5 | 2.2 | 1.5 | 2.2 |
| Moisturizer | Glycerin | 25.0 | 37.3 | 25.0 | 37.3 | 25.0 | 37.3 |
| | Propanediol | 5.0 | 7.5 | 5.0 | 7.5 | 5.0 | 7.5 |
| Preservative | Phenoxyethanol | 0.5 | 0.7 | 0.5 | 0.7 | 0.5 | 0.7 |
| pH adjuster | Citric acid | Suitable amount | | Suitable amount | | Suitable amount | |
| Chelating agent | EDTA-2Na | 0.1 | 0.1 | 0.1 | 0.1 | 0.1 | 0.1 |
| Solvent | Purified water | 54.4 | 32.0 | 54.4 | 32.0 | 54.4 | 32.0 |
| Total (% by mass) | | 100 | 100 | 100 | 100 | 100 | 100 |
| Appearance (transparency) | | A | A | A | C | - | - |
| Hardness (25°C,2Φ) | | B | B | B | C | - | - |
| Foaming properties | | A | A | C | C | - | - |
| Total volatile content (%) | | 54.4 | 32.0 | 54.4 | 32.0 | 54.4 | 32.0 |
| pH of 1% by mass aqueous solution | | 6.3 | 6.5 | 6.3 | 6.5 | 6.3 | 6.5 |

**[Table 1-2]**

| Classification | Component name | Test Example | | | |
|---|---|---|---|---|---|
| | | 1-4 | 1-4 (aging) | 1-5 | 1-5 (aging) |
| Anionic SAA | Lauryl glycol carboxylate Na | - | - | - | - |
| | Cocoyl methyl taurine Na | - | - | - | - |
| | Cocoyl methyl taurine taurine Na | - | - | - | - |
| | Lauroyl methylalanine Na | 7.5 | 11.2 | - | - |
| | Lauroyl-β-alanine Na | - | - | 7.5 | 11.2 |
| Amphoteric SAA | Cocamidopropyl betaine | 4.0 | 6.0 | 4.0 | 6.0 |
| Nonionic SAA | Cocamide methyl MEA | 2.0 | 3.0 | 2.0 | 3.0 |
| Gelling agent | Tamarind gum | 1.5 | 2.2 | 1.5 | 2.2 |
| Moisturizer | Glycerin | 25.0 | 37.3 | 25.0 | 37.3 |
| | Propanediol | 5.0 | 7.5 | 5.0 | 7.5 |
| Preservative | Phenoxyethanol | 0.5 | 0.7 | 0.5 | 0.7 |
| pH adjuster | Citric acid | Suitable amount | | Suitable amount | |
| Chelating agent | EDTA-2Na | 0.1 | 0.1 | 0.1 | 0.1 |
| Solvent | Purified water | 54.4 | 32.0 | 54.4 | 32. 0 |
| Total (% by mass) | | 100 | 100 | 100 | 100 |
| Appearance (transparency) | | A | A | B | B |
| Hardness (25°C, 2Φ) | | A | A | C | C |
| Foaming properties | | A | A | C | C |
| Total volatile content (%) | | 54.4 | 32.0 | 54.4 | 32.0 |
| pH of 1% by mass aqueous solution | | 6.3 | 6.5 | 6.3 | 6.5 |

**[Table 1-3]**

| Classificatio n | Component name | Test Example | | | | | |
|---|---|---|---|---|---|---|---|
| | | 1-6 | 1-6 (aging) | 1-7 | 1-7 (aging) | 1-8 | 1-8 (aging) |
| Anionic SAA | Lauroyl methylalanine TEA | 7.5 | 11.2 | - | - | - | - |
| | Cocoyl glutamate TEA | - | - | 7.5 | 11.2 | - | - |
| | Acyl (C12,C14)-aspartate TEA | - | - | - | - | 7.5 | 11.2 |
| | Cocoyl glycine K | - | - | - | - | - | - |
| | Cocoyl glycine Na | - | - | - | - | - | - |
| Amphoteric SAA | Cocamidopropyl betaine | 4.0 | 6.0 | 4.0 | 6.0 | 4.0 | 6.0 |
| Nonionic SAA | Cocamide methyl MEA | 2.0 | 3.0 | 2.0 | 3.0 | 2.0 | 3.0 |
| Gelling agent | Tamarind gum | 1.5 | 2.2 | 1.5 | 2.2 | 1.5 | 2.2 |
| Moisturizer | Glycerin | 25.0 | 37.3 | 25.0 | 37.3 | 25.0 | 37.3 |
| | Propanediol | 5.0 | 7.5 | 5.0 | 7.5 | 5.0 | 7.5 |
| Preservative | Phenoxyethanol | 0.5 | 0.7 | 0.5 | 0.7 | 0.5 | 0.7 |
| pH adjuster | Citric acid | Suitable amount | | Suitable amount | | Suitable amount | |
| Chelating agent | EDTA-2Na | 0.1 | 0.1 | 0.1 | 0.1 | 0.1 | 0.1 |
| Solvent | Purified water | 54.4 | 32.0 | 54.4 | 32.0 | 54.4 | 32.0 |
| Total (% by mass) | | 100 | 100 | 100 | 100 | 100 | 100 |
| Appearance (transparency) | | B | B | D | D | B | B |
| Hardness (25°C, 2Φ) | | B | B | B | B | A | A |
| Foaming properties | | C | C | C | C | B | B |
| Total volatile content (%) | | 54.4 | 32.0 | 54.4 | 32.0 | 54.4 | 32.0 |
| pH of 1% by mass aqueous solution | | 6.3 | 6.5 | 6.3 | 6.5 | 6.3 | 6.5 |

**[Table 1-4]**

| Classification | Component name | Test Example | | | |
|---|---|---|---|---|---|
| | | 1-9 | 1-9 (aging) | 1-10 | 1-10 (aging) |
| Anionic SAA | Lauroyl methylalanine TEA | - | - | - | - |
| | Cocoyl glutamate TEA | - | - | - | - |
| | Acyl (C12,C14)-aspartate TEA | - | - | - | - |
| | Cocoyl glycine K | 7.5 | 11.2 | - | - |
| | Cocoyl glycine Na | - | - | 7.5 | 11.2 |
| Amphoteric SAA | Cocamidopropyl betaine | 4.0 | 6.0 | 4.0 | 6.0 |
| Nonionic SAA | Cocamide methyl MEA | 2.0 | 3.0 | 2.0 | 3.0 |
| Gelling agent | Tamarind gum | 1.5 | 2.2 | 1.5 | 2.2 |
| Moisturizer | Glycerin | 25.0 | 37.3 | 25.0 | 37.3 |
| | Propanediol | 5.0 | 7.5 | 5.0 | 7.5 |
| Preservative | Phenoxyethanol | 0.5 | 0.7 | 0.5 | 0.7 |
| pH adjuster | Citric acid | Suitable amount | | Suitable amount | |
| Chelating agent | EDTA-2Na | 0.1 | 0.1 | 0.1 | 0.1 |
| Solvent | Purified water | 54.4 | 32.0 | 54.4 | 32.0 |
| Total (% by mass) | | 100 | 100 | 100 | 100 |
| Appearance (transparency) | | A | D | A | D |
| Hardness (25°C, 2Φ) | | A | A | A | A |
| Foaming properties | | A | A | A | A |
| Total volatile content (%) | | 54.4 | 32.0 | 54.4 | 32.0 |
| pH of 1% by mass aqueous solution | | 6.3 | 6.5 | 6.3 | 6.5 |

Tables 1-1 to 1-4 show that lauroyl methylalanine Na (Test Example 1-4) was the best among the various anionic surfactants.

Test Examples 1-2, 1-9, and 1-10 (in which cocoyl methyl taurine Na, cocoyl glycine K, and cocoyl glycine Na were incorporated, respectively), which showed good results in terms of appearance (transparency) and foaming properties during production, tend to become cloudy after aging.

Further, lauryl glycol carboxylate Na (Test Example 1-1) did not cause transparency problems; however, sufficient hardness could not be obtained during production or after aging.

Therefore, in terms of appearance (transparency), hardness, and foaming properties, it is considered that lauroyl methylalanine Na is preferred among the various anionic surfactants.

### (Test Examples 2-1 to 2-27)

Since gelation using tamarind gum had different effects on production suitability (viscosity), hardness, syneresis properties, and appearance (transparency) depending on the moisturizer used, moisturizers such as polyhydric alcohol were examined.

According to the formulations shown in Table 2-1 to Table 2-9, water, polyhydric alcohol, etc. were placed in a production pot and dissolved by heating, after which xyloglucan was added and dissolved by stirring. Thereafter, a surfactant etc. were added, and the mixture was uniformly mixed and cooled (cooling solidification step).

Because a relatively large amount of water was used as a solvent as a preparation component, after cooling, the solvent was removed by evaporation in a constant temperature and humidity chamber for 1 month to 2 months of aging period (aging step), thereby obtaining gel cleaning agents.

Next, the appearance (transparency), hardness, production suitability (viscosity), and syneresis properties were evaluated before and after the aging step. The results are shown in Table 2-1 to Table 2-9.

**[Table 2-1]**

| Classificati on | Component name | Test Example | | | | | |
|---|---|---|---|---|---|---|---|
| | | 2-1 | 2-1 (aging) | 2-2 | 2-2 (aging) | 2-3 | 2-3 (aging) |
| Anionic SAA | Lauroyl methylalanine Na | 7.5 | 19.9 | 7.5 | 14.3 | 7.5 | 11.2 |
| Amphoteric SAA | Cocamidopropyl betaine | 4.0 | 10.6 | 4.0 | 7.6 | 4.0 | 6.0 |
| Nonionic SAA | Cocamide methyl MEA | 2.0 | 5.3 | 2.0 | 3.8 | 2.0 | 3.0 |
| Gelling agent | Tamarind gum | 1.5 | 4.0 | 1.5 | 2.9 | 1.5 | 2.2 |
| Moisturizer | 1,3-Butylene glycol | 10.0 | 26.6 | 20.0 | 38.2 | 30.0 | 44.7 |
| | Propylene glycol | - | - | - | - | - | - |
| Preservative | Phenoxyethanol | 0.5 | 1.3 | 0.5 | 1.0 | 0.5 | 0.7 |
| pH adjuster | Citric acid | Suitable amount | | Suitable amount | | Suitable amount | |
| Chelating agent | EDTE-2Na | 0.1 | 0.3 | 0.1 | 0.2 | 0.1 | 0.1 |
| Solvent | Purified water | 74.4 | 32.0 | 64.4 | 32.0 | 54.4 | 32.0 |
| Total (% by mass) | | 100 | 100 | 100 | 100 | 100 | 100 |
| Appearance (transparency) | | B | C | C | D | D | D |
| Hardness (25°C, 2Φ) | | B | A | A | A | A | A |
| Production suitability | | A | - | A | - | A | - |
| Syneresis properties | | E | C | E | C | E | C |
| Total volatile content (%) | | 74.4 | 32.0 | 64.4 | 32.0 | 54.4 | 32.0 |
| pH of 1% by mass aqueous solution | | 6.3 | 6.5 | 6.3 | 6.5 | 6.3 | 6.5 |

**[Table 2-2]**

| Classificati on | Component name | Test Example | | | | | |
|---|---|---|---|---|---|---|---|
| | | 2-4 | 2-4 (aging) | 2-5 | 2-5 (aging) | 2-6 | 2-6 (aging) |
| Anionic SAA | Lauroyl methylalanine Na | 7.5 | 19.9 | 7.5 | 14.3 | 7.5 | 11.2 |
| Amphoteric SAA | Cocamidopropyl betaine | 4.0 | 10.6 | 4.0 | 7.6 | 4.0 | 6.0 |
| Nonionic SAA | Cocamide methyl MEA | 2.0 | 5.3 | 2.0 | 3.8 | 2.0 | 3.0 |
| Gelling agent | Tamarind gum | 1.5 | 4.0 | 1.5 | 2.9 | 1.5 | 2.2 |
| Moisturizer | 1,3-Butylene glycol | - | - | - | - | - | - |
| | Propylene glycol | 10.0 | 26.6 | 20.0 | 38.2 | 30.0 | 44.7 |
| Preservative | Phenoxyethanol | 0.5 | 1.3 | 0.5 | 1.0 | 0.5 | 0.7 |
| pH adjuster | Citric acid | Suitable amount | | Suitable amount | | Suitable amount | |
| Chelating agent | EDTE-2Na | 0.1 | 0.3 | 0.1 | 0.2 | 0.1 | 0.1 |
| Solvent | Purified water | 74.4 | 32.0 | 64.4 | 32.0 | 54.4 | 32.0 |
| Total (% by mass) | | 100 | 100 | 100 | 100 | 100 | 100 |
| Appearance (transparency) | | B | C | C | D | D | D |
| Hardness (25°C, 2Φ) | | B | A | A | A | A | A |
| Production suitability | | A | - | A | - | A | - |
| Syneresis properties | | C | B | C | B | C | B |
| Total volatile content (%) | | 74.4 | 32.0 | 64.4 | 32.0 | 54.4 | 32.0 |
| pH of 1% by mass aqueous solution | | 6.3 | 6.5 | 6.3 | 6.5 | 6.3 | 6.5 |

**[Table 2-3]**

| Classificati on | Component name | Test Example | | | | | |
|---|---|---|---|---|---|---|---|
| | | 2-7 | 2-7 (aging) | 2-8 | 2-8 (aging) | 2-9 | 2-9 (aging) |
| Anionic SAA | Lauroyl methylalanine Na | 7.5 | 19.9 | 7.5 | 14.3 | 7.5 | 11.2 |
| Amphoteric SAA | Cocamidopropyl betaine | 4.0 | 10.6 | 4.0 | 7.6 | 4.0 | 6.0 |
| Nonionic SAA | Cocamide methyl MEA | 2.0 | 5.3 | 2.0 | 3.8 | 2.0 | 3.0 |
| Gelling agent | Tamarind gum | 1.5 | 4.0 | 1.5 | 2.9 | 1.5 | 2.2 |
| Moisturizer | Propanediol | 10.0 | 26.6 | 20.0 | 38.2 | 30.0 | 44.7 |
| | Glycerin | - | - | - | - | - | - |
| Preservative | Phenoxyethanol | 0.5 | 1.3 | 0.5 | 1.0 | 0.5 | 0.7 |
| pH adjuster | Citric acid | Suitable amount | | Suitable amount | | Suitable amount | |
| Chelating agent | EDTE-2Na | 0.1 | 0.3 | 0.1 | 0.2 | 0.1 | 0.1 |
| Solvent | Purified water | 74.4 | 32.0 | 64.4 | 32.0 | 54.4 | 32.0 |
| Total (% by mass) | | 100 | 100 | 100 | 100 | 100 | 100 |
| Appearance (transparency) | | B | C | B | D | D | D |
| Hardness (25°C, 2Φ) | | B | A | A | A | A | A |
| Production suitability | | A | - | A | - | A | - |
| Syneresis properties | | C | B | C | B | C | B |
| Total volatile content (%) | | 74.4 | 32.0 | 64.4 | 32.0 | 54.4 | 32.0 |
| pH of 1% by mass aqueous solution | | 6.3 | 6.5 | 6.3 | 6.5 | 6.3 | 6.5 |

**[Table 2-4]**

| Classificati on | Component name | Test Example | | | | | |
|---|---|---|---|---|---|---|---|
| | | 2-10 | 2-10 (aging) | 2-11 | 2-11 (aging) | 2-12 | 2-12 (aging) |
| Anionic SAA | Lauroyl methylalanine Na | 7.5 | 19.9 | 7.5 | 14.3 | 7.5 | 11.2 |
| Amphoteric SAA | Cocamidopropyl betaine | 4.0 | 10.6 | 4.0 | 7.6 | 4.0 | 6.0 |
| Nonionic SAA | Cocamide methyl MEA | 2.0 | 5.3 | 2.0 | 3.8 | 2.0 | 3.0 |
| Gelling agent | Tamarind gum | 1.5 | 4.0 | 1.5 | 2.9 | 1.5 | 2.2 |
| Moisturizer | Propanediol | - | - | - | - | - | - |
| | Glycerin | 10.0 | 26.6 | 20.0 | 38.2 | 30.0 | 44.7 |
| Preservative | Phenoxyethanol | 0.5 | 1.3 | 0.5 | 1.0 | 0.5 | 0.7 |
| pH adjuster | Citric acid | Suitable amount | | Suitable amount | | Suitable amount | |
| Chelating agent | EDTE-2Na | 0.1 | 0.3 | 0.1 | 0.2 | 0.1 | 0.1 |
| Solvent | Purified water | 74.4 | 32.0 | 64.4 | 32.0 | 54.4 | 32.0 |
| Total (% by mass) | | 100 | 100 | 100 | 100 | 100 | 100 |
| Appearance (transparency) | | - | - | A | B | A | A |
| Hardness (25°C, 2Φ) | | - | - | C | B | B | A |
| Production suitability | | - | - | A | - | A | - |
| Syneresis properties | | - | - | B | A | B | A |
| Total volatile content (%) | | 74.4 | 32.0 | 64.4 | 32.0 | 54.4 | 32.0 |
| pH of 1% by mass aqueous solution | | 6.3 | 6.5 | 6.3 | 6.5 | 6.3 | 6.5 |

**[Table 2-5]**

| Classificati on | Component name | Test Example | | | | | |
|---|---|---|---|---|---|---|---|
| | | 2-13 | 2-13 (aging) | 2-14 | 2-14 (aging) | 2-15 | 2-15 (aging) |
| Anionic SAA | Lauroyl methylalanine Na | 7.5 | 19.9 | 7.5 | 14.3 | 7.5 | 11.2 |
| Amphoteric SAA | Cocamidopropyl betaine | 4.0 | 10.6 | 4.0 | 7.6 | 4.0 | 6.0 |
| Nonionic SAA | Cocamide methyl MEA | 2.0 | 5.3 | 2.0 | 3.8 | 2.0 | 3.0 |
| Gelling agent | Tamarind gum | 1.5 | 4.0 | 1.5 | 2.9 | 1.5 | 2.2 |
| Moisturizer | Trehalose | 10.0 | 26.6 | 20.0 | 38.2 | 30.0 | 44.7 |
| | Diglycerin | - | - | - | - | - | - |
| Preservative | Phenoxyethanol | 0.5 | 1.3 | 0.5 | 1.0 | 0.5 | 0.7 |
| pH adjuster | Citric acid | Suitable amount | | Suitable amount | | Suitable amount | |
| Chelating agent | EDTE-2Na | 0.1 | 0.3 | 0.1 | 0.2 | 0.1 | 0.1 |
| Solvent | Purified water | 74.4 | 32.0 | 64.4 | 32.0 | 54.4 | 32.0 |
| Total (% by mass) | | 100 | 100 | 100 | 100 | 100 | 100 |
| Appearance (transparency) | | - | - | - | - | A | A |
| Hardness (25°C,2Φ) | | - | - | - | - | C | B |
| Production suitability | | - | - | A | - | A | - |
| Syneresis properties | | - | - | - | - | B | A |
| Total volatile content (%) | | 74.4 | 32.0 | 64.4 | 32.0 | 54.4 | 32.0 |
| pH of 1% by mass aqueous solution | | 6.3 | 6.5 | 6.3 | 6.5 | 6.3 | 6.5 |

**[Table 2-6]**

| Classificati on | Component name | Test Example | | | | | |
|---|---|---|---|---|---|---|---|
| | | 2-16 | 2-16 (aging) | 2-17 | 2-17 (aging) | 2-18 | 2-18 (aging) |
| Anionic SAA | Lauroyl methylalanine Na | 7.5 | 19.9 | 7.5 | 14.3 | 7.5 | 11.2 |
| Amphoteric SAA | Cocamidopropyl betaine | 4.0 | 10.6 | 4.0 | 7.6 | 4.0 | 6.0 |
| Nonionic SAA | Cocamide methyl MEA | 2.0 | 5.3 | 2.0 | 3.8 | 2.0 | 3.0 |
| Gelling agent | Tamarind gum | 1.5 | 4.0 | 1.5 | 2.9 | 1.5 | 2.2 |
| Moisturizer | Trehalose | - | - | - | - | - | - |
| | Diglycerin | 10.0 | 26.6 | 20.0 | 38.2 | 30.0 | 44.7 |
| Preservative | Phenoxyethanol | 0.5 | 1.3 | 0.5 | 1.0 | 0.5 | 0.7 |
| pH adjuster | Citric acid | Suitable amount | | Suitable amount | | Suitable amount | |
| Chelating agent | EDTE-2Na | 0.1 | 0.3 | 0.1 | 0.2 | 0.1 | 0.1 |
| Solvent | Purified water | 74.4 | 32.0 | 64.4 | 32.0 | 54.4 | 32.0 |
| Total (% by mass) | | 100 | 100 | 100 | 100 | 100 | 100 |
| Appearance (transparency) | | - | - | A | B | B | B |
| Hardness (25°C, 2Φ) | | - | - | B | B | A | A |
| Production suitability | | - | - | B | - | C | - |
| Syneresis properties | | - | - | B | A | B | A |
| Total volatile content (%) | | 74.4 | 32.0 | 64.4 | 32.0 | 54.4 | 32.0 |
| pH of 1% by mass aqueous solution | | 6.3 | 6.5 | 6.3 | 6.5 | 6.3 | 6.5 |

**[Table 2-7]**

| Classificati on | Component name | Test Example | | | | | |
|---|---|---|---|---|---|---|---|
| | | 2-19 | 2-19 (aging) | 2-20 | 2-20 (aging) | 2-21 | 2-21 (aging) |
| Anionic SAA | Lauroyl methylalanine Na | 7.5 | 19.9 | 7.5 | 14.3 | 7.5 | 11.2 |
| Amphoteric SAA | Cocamidopropyl betaine | 4.0 | 10.6 | 4.0 | 7.6 | 4.0 | 6.0 |
| Nonionic SAA | Cocamide methyl MEA | 2.0 | 5.3 | 2.0 | 3.8 | 2.0 | 3.0 |
| Gelling agent | Tamarind gum | 1.5 | 4.0 | 1.5 | 2.9 | 1.5 | 2.2 |
| Moisturizer | Xylitol | 10.0 | 26.6 | 20.0 | 38.2 | 30.0 | 44.7 |
| | Sorbitol | - | - | - | - | - | - |
| Preservative | Phenoxyethanol | 0.5 | 1.3 | 0.5 | 1.0 | 0.5 | 0.7 |
| pH adjuster | Citric acid | Suitable amount | | Suitable amount | | Suitable amount | |
| Chelating agent | EDTE-2Na | 0.1 | 0.3 | 0.1 | 0.2 | 0.1 | 0.1 |
| Solvent | Purified water | 74.4 | 32.0 | 64.4 | 32.0 | 54.4 | 32.0 |
| Total (% by mass) | | 100 | 100 | 100 | 100 | 100 | 100 |
| Appearance (transparency) | | - | - | A | B | A | A |
| Hardness (25**°C**, 2Φ) | | - | - | C | B | C | B |
| Production suitability | | - | - | A | - | A | - |
| Syneresis properties | | - | - | B | A | B | A |
| Total volatile content (%) | | 74.4 | 32.0 | 64.4 | 32.0 | 54.4 | 32.0 |
| pH of 1% by mass aqueous solution | | 6.3 | 6.5 | 6.3 | 6.5 | 6.3 | 6.5 |

**[Table 2-8]**

| Classificati on | Component name | Test Example | | | | | |
|---|---|---|---|---|---|---|---|
| | | 2-22 | 2-22 (aging) | 2-23 | 2-23 (aging) | 2-24 | 2-24 (aging) |
| Anionic SAA | Lauroyl methylalanine Na | 7.5 | 19.9 | 7.5 | 14.3 | 7.5 | 11.2 |
| Amphoteric SAA | Cocamidopropyl betaine | 4.0 | 10.6 | 4.0 | 7.6 | 4.0 | 6.0 |
| Nonionic SAA | Cocamide methyl MEA | 2.0 | 5.3 | 2.0 | 3.8 | 2.0 | 3.0 |
| Gelling agent | Tamarind gum | 1.5 | 4.0 | 1.5 | 2.9 | 1.5 | 2.2 |
| Moisturizer | Xylitol | - | - | - | - | - | - |
| | Sorbitol | 10.0 | 26.6 | 20.0 | 38.2 | 30.0 | 44.7 |
| Preservative | Phenoxyethanol | 0.5 | 1.3 | 0.5 | 1.0 | 0.5 | 0.7 |
| pH adjuster | Citric acid | Suitable amount | | Suitable amount | | Suitable amount | |
| Chelating agent | EDTE-2Na | 0.1 | 0.3 | 0.1 | 0.2 | 0.1 | 0.1 |
| Solvent | Purified water | 74.4 | 32.0 | 64.4 | 32.0 | 54.4 | 32.0 |
| Total (% by mass) | | 100 | 100 | 100 | 100 | 100 | 100 |
| Appearance (transparency) | | - | - | A | D | A | D |
| Hardness (25**°C**, 2Φ) | | - | - | C | B | C | B |
| Production suitability | | - | - | B | - | C | - |
| Syneresis properties | | - | - | B | A | B | A |
| Total volatile content (%) | | 74.4 | 32.0 | 64.4 | 32.0 | 54.4 | 32.0 |
| pH of 1% by mass aqueous solution | | 6.3 | 6.5 | 6.3 | 6.5 | 6.3 | 6.5 |

**[Table 2-9]**

| Classificati on | Component name | Test Example | | | | | |
|---|---|---|---|---|---|---|---|
| | | 2-25 | 2-25 (aging) | 2-26 | 2-26 (aging) | 2-27 | 2-27 (aging) |
| Anionic SAA | Lauroyl methylalanine Na | 7.5 | 19.9 | 7.5 | 14.3 | 7.5 | 11.2 |
| Amphoteric SAA | Cocamidopropyl betaine | 4.0 | 10.6 | 4.0 | 7.6 | 4.0 | 6.0 |
| Nonionic SAA | Cocamide methyl MEA | 2.0 | 5.3 | 2.0 | 3.8 | 2.0 | 3.0 |
| Gelling agent | Tamarind gum | 1.5 | 4.0 | 1.5 | 2.9 | 1.5 | 2.2 |
| Moisturizer | Sucrose | 10.0 | 26.6 | 20.0 | 38.2 | 30.0 | 44.7 |
| Preservative | Phenoxyethanol | 0.5 | 1.3 | 0.5 | 1.0 | 0.5 | 0.7 |
| pH adjuster | Citric acid | Suitable amount | | Suitable amount | | Suitable amount | |
| Chelating agent | EDTE-2Na | 0.1 | 0.3 | 0.1 | 0.2 | 0.1 | 0.1 |
| Solvent | Purified water | 74.4 | 32.0 | 64.4 | 32.0 | 54.4 | 32.0 |
| Total (% by mass) | | 100 | 100 | 100 | 100 | 100 | 100 |
| Appearance (transparency) | | - | - | - | - | A | A |
| Hardness (25**°C**, 2Φ) | | - | - | - | - | C | B |
| Production suitability | | A | - | A | - | B | - |
| Syneresis properties | | - | - | B | A | B | A |
| Total volatile content (%) | | 74.4 | 32.0 | 64.4 | 32.0 | 54.4 | 32.0 |
| pH of 1% by mass aqueous solution | | 6.3 | 6.5 | 6.3 | 6.5 | 6.3 | 6.5 |

Table 2-1 to Table 2-9 show that in Test Example 2-1 to Test Example 2-9, in which 1,3-butylene glycol, propylene glycol, and propanediol are used, solidification occur when incorporating about 10% by mass of each component, and the hardness tends to increase as the incorporation amount increases.

On the other hand, the appearance is translucent to opaque, and syneresis is also observed (it was found that among the three raw materials, propanediol tended to have good transparency and syneresis properties).

As for Test Examples 2-10 to 2-27, in which glycerin, diglycerin, trehalose, xylitol, sorbitol, or sucrose was used, 20% by mass of each component (30% by mass or more of sucrose) was required for solidification. Compared to 1,3-butylene glycol, propylene glycol, and propanediol, the hardness tended to be low; however, there was less syneresis, and transparency was good. Among these, Test Example 2-11 and Test Example 2-12, in which 20% by mass or more of glycerin was incorporated, were good in terms of hardness, syneresis properties, and appearance.

### (Test Examples 3-1 to 3-6)

The optimal amounts of glycerin and propanediol to be incorporated, and the incorporation ratio of the two were examined.

According to the formulations shown in Table 3-1 to Table 3-4, water, polyhydric alcohol, etc. were placed in a production pot and dissolved by heating, after which xyloglucan was added and dissolved by stirring. Thereafter, a surfactant etc. were added, and the mixture was uniformly mixed and cooled (cooling solidification step).

Because a relatively large amount of water was used as a solvent as a preparation component, after cooling, the solvent was removed by evaporation in a constant temperature and humidity chamber for 1 month to 2 months of aging period (aging step), thereby obtaining gel cleaning agents.

Next, the appearance (transparency), hardness, production suitability, and syneresis properties were evaluated before and after the aging step, and the total amount of glycerin and propanediol, and the proportion of glycerin were calculated. The results are shown in Tables 3-1 to 3-4.

**[Table 3-1]**

| Classificati on | Component name | Test Example | | | | | |
|---|---|---|---|---|---|---|---|
| | | 2-5 | 2-5 (aging) | 2-6 | 2-6 (aging) | 3-1 | 3-1 (aging) |
| Anionic SAA | Lauroyl methylalanine Na | 7.5 | 14.3 | 7.5 | 11.2 | 7.5 | 9.2 |
| Amphoteric SAA | Cocamidopropyl betaine | 4.0 | 7.6 | 4.0 | 6.0 | 4.0 | 6. 0 |
| Nonionic SAA | Cocamide methyl MEA | 2.0 | 3.8 | 2.0 | 3.0 | 2.0 | 3.0 |
| Gelling agent | Tamarind gum | 1.5 | 2.9 | 1.5 | 2.2 | 1.5 | 2.2 |
| Moisturizer | Glycerin | 20.0 | 38.2 | 30.0 | 44.7 | 40.0 | 59.6 |
| | Propanediol | - | - | - | - | - | - |
| Preservative | Phenoxyethanol | 0.5 | 1.0 | 0.5 | 0.7 | 0.5 | 0.7 |
| pH adjuster | Citric acid | Suitable amount | | Suitable amount | | Suitable amount | |
| Chelating agent | EDTE-2Na | 0.1 | 0.2 | 0.1 | 0.1 | 0.1 | 0.1 |
| Solvent | Purified water | 64.4 | 32.0 | 54.4 | 32.0 | 44.4 | 19.1 |
| Total (% by mass) | | 100 | 100 | 100 | 100 | 100 | 100 |
| Appearance (transparency) | | A | B | A | A | A | A |
| Hardness (25**°C**, 2Φ) | | C | B | B | A | B | A |
| Production suitability | | A | - | A | - | B | - |
| Syneresis properties | | B | A | B | A | B | A |
| Total volatile content (%) | | 64.4 | 32.0 | 54.4 | 32.0 | 44.4 | 32.0 |
| Total amount of glycerin and propanediol (% by mass) | | 20.0 | 38.2 | 30.0 | 44.7 | 40.0 | 59.6 |
| Proportion of glycerin (% by mass) | | 100 | 100 | 100 | 100 | 100 | 100 |
| pH of 1% by mass aqueous solution | | 6.3 | 6.5 | 6.3 | 6.5 | 6.3 | 6.5 |

**[Table 3-2]**

| Classificati on | Component name | Test Example | | | | | |
|---|---|---|---|---|---|---|---|
| | | 3-2 | 3-2 (aging) | 2-5 | 2-5 (aging) | 2-6 | 2-6 (aging) |
| Anionic SAA | Lauroyl methylalanine Na | 7.5 | 7.8 | 7.5 | 14.3 | 7.5 | 11.2 |
| Amphoteric SAA | Cocamidopropyl betaine | 4.0 | 4.1 | 4.0 | 7.6 | 4.0 | 6.0 |
| Nonionic SAA | Cocamide methyl MEA | 2.0 | 2.1 | 2.0 | 3.8 | 2.0 | 3.0 |
| Gelling agent | Tamarind gum | 1.5 | 1.6 | 1.5 | 2.9 | 1.5 | 2.2 |
| Moisturizer | Glycerin | 50.0 | 51.8 | 20.0 | 38.2 | 30.0 | 44.7 |
| | Propanediol | - | - | - | - | - | - |
| Preservative | Phenoxyethanol | 0.5 | 0.5 | 0.5 | 1.0 | 0.5 | 0.7 |
| pH adjuster | Citric acid | Suitable amount | | Suitable amount | | Suitable amount | |
| Chelating agent | EDTE-2Na | 0.1 | 0.1 | 0.1 | 0.2 | 0.1 | 0.1 |
| Solvent | Purified water | 34.4 | 32.0 | 64.4 | 32.0 | 54.4 | 32.0 |
| Total (% by mass) | | 100 | 100 | 100 | 100 | 100 | 100 |
| Appearance (transparency) | | - | - | A | B | A | A |
| Hardness (25**°C**, 2Φ) | | - | - | C | B | B | A |
| Production suitability | | D | - | A | - | A | - |
| Syneresis properties | | - | - | B | A | B | A |
| Total volatile content (%) | | 34.4 | 32.0 | 64.4 | 32.0 | 54.4 | 32.0 |
| Total amount of glycerin and propanediol (% by mass) | | 50.0 | 51.8 | 20.0 | 38.2 | 30.0 | 44.7 |
| Proportion of glycerin (% by mass) | | 100 | 100 | 100 | 100 | 100 | 100 |
| pH of 1% by mass aqueous solution | | 6.3 | 6.5 | 6.3 | 6.5 | 6.3 | 6.5 |

**[Table 3-3]**

| Classificati on | Component name | Test Example | | | | | |
|---|---|---|---|---|---|---|---|
| | | 3-3 | 3-3 (aging) | 3-4 | 3-4 (aging) | 3-5 | 3-5 (aging) |
| Anionic SAA | Lauroyl methylalanine Na | 7.5 | 11.2 | 7.5 | 11.2 | 7.5 | 9.2 |
| Amphoteric SAA | Cocamidopropyl betaine | 4.0 | 6.0 | 4.0 | 6.0 | 4.0 | 4.9 |
| Nonionic SAA | Cocamide methyl MEA | 2.0 | 3.0 | 2.0 | 3.0 | 2.0 | 2.4 |
| Gelling agent | Tamarind gum | 1.5 | 2.2 | 1.5 | 2.2 | 1.5 | 1.8 |
| Moisturizer | Glycerin | 20.0 | 29.8 | 25.0 | 37.3 | 30.0 | 36.7 |
| | Propanediol | 10.0 | 14.9 | 5.0 | 7.5 | 10.0 | 12.2 |
| Preservative | Phenoxyethanol | 0.5 | 0.7 | 0.5 | 0.7 | 0.5 | 0.6 |
| pH adjuster | Citric acid | Suitable amount | | Suitable amount | | Suitable amount | |
| Chelating agent | EDTE-2Na | 0.1 | 0.1 | 0.1 | 0.1 | 0.1 | 0.1 |
| Solvent | Purified water | 54.4 | 32.0 | 54.4 | 32.0 | 44.4 | 32.0 |
| Total (% by mass) | | 100 | 100 | 100 | 100 | 100 | 100 |
| Appearance (transparency) | | B | B | A | A | B | B |
| Hardness (25**°C**, 2Φ) | | A | A | A | A | A | A |
| Production suitability | | A | - | A | - | A | - |
| Syneresis properties | | B | A | B | A | B | A |
| Total volatile content (%) | | 54.4 | 32.0 | 54.4 | 32.0 | 44.4 | 32.0 |
| Total amount of glycerin and propanediol (% by mass) | | 30.0 | 44.7 | 30.0 | 44.7 | 40.0 | 48.9 |
| Proportion of glycerin (% by mass) | | 66.7 | 66.7 | 83.3 | 83.3 | 75.0 | 75.0 |
| pH of 1% by mass aqueous solution | | 6.3 | 6.5 | 6.3 | 6.5 | 6.3 | 6.5 |

**[Table 3-4]**

| Classification | Component name | Test Example | |
|---|---|---|---|
| | | 3-6 | 3-6 (aging) |
| Anionic SAA | Lauroyl methylalanine Na | 7.5 | 9.2 |
| Amphoteric SAA | Cocamidopropyl betaine | 4.0 | 4.9 |
| Nonionic SAA | Cocamide methyl MEA | 2.0 | 2.4 |
| Gelling agent | Tamarind gum | 1.5 | 1.8 |
| Moisturizer | Glycerin | 35.0 | 42.8 |
| | Propanediol | 5.0 | 6.1 |
| Preservative | Phenoxyethanol | 0.5 | 0.6 |
| pH adjuster | Citric acid | Suitable amount | |
| Chelating agent | EDTE-2Na | 0.1 | 0.1 |
| Solvent | Purified water | 44.4 | 32.0 |
| Total (% by mass) | | 100 | 100 |
| Appearance (transparency) | | A | A |
| Hardness (25**°C**, 2Φ) | | A | A |
| Production suitability | | A | - |
| Syneresis properties | | B | A |
| Total volatile content (%) | | 44.4 | 32.0 |
| Total amount of glycerin and propanediol (% by mass) | | 40.0 | 48.9 |
| Proportion of glycerin (% by mass) | | 87.5 | 87.5 |
| pH of 1% by mass aqueous solution | | 6.3 | 6.5 |

Table 3-1 and Table 3-4 show that in Test Example 3-2, 40% by mass or more of solvent (water) is required during production because incorporating 50% by mass of glycerin makes production difficult due to thickening.

Therefore, in terms of production suitability, the amount of glycerin is considered to be preferably within the range of 20% by mass to 40% by mass.

In Test Example 3-3 and Test Example 3-5, an increase in hardness was observed due to the incorporation of propanediol; however, in Test Example 3-3 and Test Example 3-5, when 10% by mass or more of propanediol is incorporated, the appearance tends to become opaque as in the case of single incorporation.

From the above, in order to obtain a transparent gel cleaning agent with desirable hardness by incorporating glycerin and propanediol, it is preferable that the mass of glycerin in the total amount of glycerin and propanediol is 80% by mass or more.

Next, a suitable water content in the gel cleaning agent after the aging step was examined. The results are shown in Table 4.

Test Examples 3-4 (aging 1) to (aging 5) in Table 4 indicate different aging periods (1 week to 3 months of aging periods, the aging period differs by at least 1 day between each aging step of (aging 1) to (aging 5)), and the aging period is in the order of (aging 1) < (aging 2)

### < (aging 3) < (aging 4) < (aging 5).

**[Table 4]**

| Classificati on | Component name | Test Example | | | | | |
|---|---|---|---|---|---|---|---|
| | | 3-4 | 3-4 (aging1) | 3-4 (aging2) | 3-4 (aging3) | 3-4 (aging4) | 3-4 (aging5) |
| Amoniac SAA | Lauroyl methylalanine Na | 7.5 | 9.9 | 10.7 | 11.5 | 12.3 | 13.2 |
| Amphoteric SAA | Cocamidopropyl betaine | 4.0 | 5.3 | 5.7 | 6.1 | 6.6 | 7.0 |
| Nonionic SAA | Cocamide methy MEA | 2.0 | 2.6 | 2.9 | 3.1 | 3.3 | 3.5 |
| Gelling agent | Tamarind gum | 1.5 | 2.0 | 2.1 | 2.3 | 2.5 | 2.6 |
| Moisturizer | Glycerin | 25.0 | 32.9 | 35.6 | 38.4 | 41.1 | 43.9 |
| | Propanediol | 5.0 | 6.6 | 7.1 | 7.7 | 8.2 | 8.8 |
| Preservative | Phenoxye thanol | 0.5 | 0.7 | 0.7 | 0.8 | 0.8 | 0.9 |
| pH adjuster | Citric acid | Suitable amount | | | | | |
| Chelating agent | EDTE-2Na | 0.1 | 0.1 | 0.1 | 0.2 | 0.2 | 0.2 |
| Solvent | Purified water | 54.4 | 40.0 | 35.0 | 30.0 | 25.0 | 20.0 |
| Total (% by mass) | | 100 | 100 | 100 | 100 | 100 | 100 |
| Appearance (transparency) | | A | A | A | A | A | A |
| Hardness (25**°C**, 2Φ) | | A | A | A | A | A | A |
| Production suitability | | A | - | - | - | - | - |
| Syneresis properties | | B | B | A | A | A | A |
| Usability | | B | B | A | A | B | B |
| Total volatile content (%) | | 54.4 | 40.0 | 35.0 | 30.0 | 25.0 | 20.0 |
| pH of 1% by mass aqueous solutin | | 6.3 | 6.3 | 6.3 | 6.5 | 6.5 | 6.5 |

Table 4 shows that after the aging step was performed, an increase in hardness and a decrease in syneresis properties were observed.

In Test Example 3-4 (aging 2) and Test Example 3-4 (aging 3), in which aging was performed until the water content reached 30% by mass to 35% by mass, the appearance (transparency), hardness, syneresis properties, and usability were all excellent.

In Test Example 3-4 (aging 1), in which aging was performed until the water content reached 40% by mass, the appearance (transparency) and hardness were excellent; however, tearing or blistering was observed during use, and syneresis was slightly observed as well.

In Test Example 3-4 (aging 4) and Test Example 3-4 (aging 5), in which aging was performed until the water content reached 25% by mass or less, the appearance (transparency) and hardness were excellent; however, there was less dissolving loss, and stickiness was observed during use.

Therefore, it was revealed that in order to obtain a gel cleaning agent without tearing, blistering, or stickiness during use, with moderate dissolving loss, and without syneresis, it was necessary to perform aging until the water content reached 30% by mass to 35% by mass in the composition.

(Test Examples 4-1 to 4-2)

Next, the necessity of the aging step was examined.

In Test Examples 4-1 and 4-2, from Table 4, with the same amounts of formulations as those of Test Examples 3-4 (aging 2) and (aging 3), which were particularly excellent in appearance (transparency), hardness, syneresis properties, and usability, each component was dissolved by heating or stirring, followed by cooling, without performing the aging step, for production. The results are shown in Table 5.

**[Table 5]**

| Classificati on | Component name | Test Example | | | | |
|---|---|---|---|---|---|---|
| | | 3-4 | 3-4 (aging2) | 3-4 (aging3) | 4-1 (no aging) | 4-2 (no aging) |
| Anionic SAA | Lauroyl methylalanine Na | 7.5 | 10.7 | 11.5 | 10.7 | 11.5 |
| Amphoteric SAA | Cocamidopropyl betaine | 4.0 | 5.7 | 6.1 | 5.7 | 6.1 |
| Nonionic SAA | Cocamide methyl MEA | 2.0 | 2.9 | 3.1 | 2.9 | 3.1 |
| Gelling agent | Tamarind gum | 1.5 | 2.1 | 2.3 | 2.1 | 2.3 |
| Moisturizer | Glycerin | 25 | 35.6 | 38.4 | 35.6 | 38.4 |
| | Propanediol | 5 | 7.1 | 7.7 | 7.1 | 7.7 |
| Preservative | Phenoxyethanol | 0.5 | 0.7 | 0.8 | 0.7 | 0.8 |
| pH adjuster | Citric acid | Suitable amount | | | | |
| Chelating agent | EDTE-2Na | 0.1 | 0.1 | 0.2 | 0.1 | 0.2 |
| Solvent | Purified water | 54.4 | 35.0 | 30.0 | 35.0 | 30.0 |
| Total (% by mass) | | 100 | 100 | 100 | 100 | 100 |
| Appearance (transparency) | | A | A | A | - | - |
| Hardness (25**°C**, 2Φ) | | A | A | A | - | - |
| Production suitability | | A | - | - | - | - |
| Syneresis properties | | B | A | A | - | - |
| Usability | | B | A | A | - | - |
| Total volatile content (%) | | 54.4 | 35.0 | 30.0 | 35.0 | 30.0 |
| pH of 1% by mass aqueous solution | | 6.3 | 6.3 | 6.5 | - | - |

Table 5 shows that in Test Example 4-1 and Test Example 4-2, according the same formulations as those of Test Example 3-4 (aging 2) and Test Example 3-4 (aging 3)

(the same formulations after the aging step), respectively, each component was dissolved by heating or stirring, followed by cooling, without performing the aging step, to try to produce gel cleaning agents, and as a result, in both Test Example 4-1 and Test Example 4-2, the water content was low and viscosity was too high, so that gel cleaning agents could not be produced (all evaluation items could not be measured). Further, as described above, in terms of production suitability, the amount of water incorporated during production is 40% by mass to 60% by mass in the composition.

These results revealed that according to the gel cleaning agent of the present invention and the method for producing the gel cleaning agent, a gel cleaning agent with excellent appearance (transparency), hardness, syneresis properties, and usability could be obtained when the amount of water incorporated during production was 40% by mass to 60% by mass in the composition, and when each component was heated and dissolved by stirring, followed by cooling, after which the aging step was performed.

## Claims

1. A gel cleaning agent comprising:
(A) 0.8% by mass to 4.5% by mass of xyloglucan,
(B) 7.5% by mass to 22.5% by mass of sodium lauroyl methylalanine,
(C) 40% by mass to 50% by mass of polyhydric alcohol, and
(D) 30% by mass to 35% by mass of water;
wherein the polyhydric alcohol (C) comprises glycerin, and the amount of glycerin in the polyhydric alcohol is 80% by mass or more, and
wherein the gel cleaning agent has a hardness at 25°C of 300 or more as measured by a rheometer.

2. The gel cleaning agent according to claim 1, comprising:
(A) 1% by mass to 3% by mass of xyloglucan,
(B) 10% by mass to 15% by mass of sodium lauroyl methylalanine, and
(C) 42% by mass to 47% by mass of polyhydric alcohol.

3. The gel cleaning agent according to claim 1 or 2, wherein a 1% by mass aqueous solution of the gel cleaning agent has a pH of 7.5 or less.

4. The gel cleaning agent according to any one of claims 1 to 3, wherein the polyhydric alcohol (C) further comprises propanediol.

5. The gel cleaning agent according to claim 4, wherein the content of propanediol is 6% by mass to 9% by mass.

6. The gel cleaning agent according to any one of claims 1 to 5, which is transparent.

7. A method for producing the gel cleaning agent according to any one of claims 1 to 6, the method comprising:
a cooling solidification step of cooling and solidifying a composition in which (A') 0.5% by mass to 3% by mass of xyloglucan, (B') 5% by mass to 15% by mass of sodium lauroyl methylalanine, (C') 20% by mass to 40% by mass of polyhydric alcohol, and (D') 40% by mass to 60% by mass of water are dissolved, and
an aging step of aging a gel composition after cooling and solidification in a constant temperature and humidity chamber for one week or more to remove a solvent by evaporation from the gel composition;
wherein the polyhydric alcohol (C') comprises glycerin, and the amount of glycerin in the polyhydric alcohol is 80% by mass or more.

8. The method for producing the gel cleaning agent according to claim 7, wherein the polyhydric alcohol (C') further comprises propanediol.

9. The method for producing the gel cleaning agent according to claim 8, wherein the content of propanediol is 5% by mass to 10% by mass.
